Europäisches Patentamt

European Patent Office

Office européen des brevets

(11) Publication number: **0 229 620 B1**

# EUROPEAN PATENT SPECIFICATION

(45) Date of publication of patent specification: **10.06.92**   (51) Int. Cl.5: **A61B 17/22**, A61B 17/32

(21) Application number: **87100057.6**

(22) Date of filing: **05.01.87**

(54) **Transluminal microdissection device.**

(30) Priority: **06.01.86 US 816190**

(43) Date of publication of application:
**22.07.87 Bulletin 87/30**

(45) Publication of the grant of the patent:
**10.06.92 Bulletin 92/24**

(84) Designated Contracting States:
**BE DE FR GB IT NL SE**

(56) References cited:
**EP-A- 0 025 645**
**EP-A- 0 086 048**

(73) Proprietor: **Heart Technology, Inc.**
**Park 140 2515 140th Avenue, N.E.**
**Bellevue, Washington 98005(US)**

(72) Inventor: **Auth, David Christopher**
**1555 132nd Avenue, NE**
**Bellevue Washington(US)**

(74) Representative: **Vossius & Partner**
**Siebertstrasse 4 P.O. Box 86 07 67**
**W-8000 München 86(DE)**

## Description

The present invention relates to a mechanical device which is used in medical applications and which is capable of differentially cutting abnormal deposits from within a patient's vessels.

U.S. Patent No. 4,445,509 entitled METHOD AND APPARATUS FOR REMOVAL OF ENCLOSED ABNORMAL DEPOSITS which issued to David C. Auth on May 1, 1984 describes a rotary mechanical system for differentially cutting relatively hard intravascular deposits while sparing relatively soft, normal tissue. In the device described in that patent, a hollow channel was used for suction removal of debris generated during the cutting process in order to prevent the debris from acting as the nucleus for thrombogenesis or from occluding smaller vascular channels and thereby inhibiting the normal flow of life sustaining blood.

Suctioning of debris may not recover all of the cutting products if vascular flow is present in the artery being treated, since fluid motion at the cutting tip will immediately carry some debris downstream. U.S. Patent No. 4,207,874 entitled LASER TUNNELLING DEVICE which issued to D.S. Choy on June 17, 1980 describes an apparatus which removes intravascular deposits by using a laser to vaporize intravascular obstractions. When laser energy is used to vaporize debris, the laser may provide suffcent energy to release each constituent molecule from the host lattice or it may produce gaseous products within the solid matrix, thereby causing a rupture of the matrix and the release of smaller constituent particles of the mass. In the former case, the amount of energy required to uncouple each individual molecule is relatively large due to the binding energy of each molecule and to the large number of molecules per unit volume of obstructing mass. In the latter case, the released particles can be relatively large and may be capable of obstructing smaller vascular branches distal to the site of the treated obstruction.

In U.S. Patent No, 4,445,509, referred to above, the preferential cutting of hard deposits vis-a-vis soft normal tissue is a desirable feature. Unfortunately, harmful obstructing deposits can, on occasion, be soft. Frequently, such soft occluding deposits are also lacking in physical toughness, i.e., they lack the ability to recover after deformation. Muscular tissue tends to be rather tough and to be able to recover after severe elastic deformation. Thus, an additional physical property which may be considered for differentiating the cutting efficacy of a particular device is its ability to distinguish between soft (compliant), tough tissue, which will not break up as a result of local deformation, and soft, weak tissue, which will break up under local deformation. As taught in U.S. Patent No. 4,445,509, the differential cutting action derives from the ability of soft tissue to "dive" out of the way before it is caught in front of the cutting edge and cleaved off. The process of "diving" implies deformation which can decimate soft, weak tissue without damaging soft, tough tissue. However, even soft, tough tissue can be cleaved if the rate at which the deformation required to escape cleavage exceeds the speed with which the tissue can move given its own inertia. Thus, increasing the surface speed of the cutting edge can eventually result in the ability to cleave soft, tough tissue. This distinction can be useful when it is desirable to cleave obstructive tissue masses which are soft and weak or soft and tough. Depending upon the local vessel anatomy, some damage to normal vessel endothelium or media may occur, and although less than desirable, that may well be a price worth paying to relieve the underlying obstructive condition. Since damage to endothelium and media occurs routinely in surgical vessel grafts which subsequently re-endothelize, the prognosis for rehealing of intima and media damaged adjacent to removed pathological material is good. Administration of drugs which suppress normal clotting may be required to inhibit thrombosis at the damage site during and after treatment.

When intravascular obstructions have a fibrous structure, there is a tendency to turn up a "scab" of material at the base of the cutting zone. Such scabs grow in size with additional cutting rather than being clipped off. They can present a problem if left within the artery, as they may flop across the arterial lumen and obstruct flow or they may become a nucleating site for thrombogenesis or regrowth of atheroma.

The object is to provide a transluminal microdissection device which is useful in a wide variety of applications.

This object is achieved by the features of independent claim 1. The subclaims are directed to preferred embodiments of the invention.

It has been found that tiny cutting surfaces which act as shovels operating at surface speeds of approx. 12.2 m/s (about 40 feet per second) can strip off microscopic divots before a scab can grow to appreciable size. These tiny shovels are preferrably comprised of fragments of diamond crystal or grit. Other sharp grit could be used, but diamond is inexpensive in this format and provides good wear characteristics. When these crystalline fragments (shovels) are very small in size, they necessarily generate very small debris fragments. If the debris tissue fragments are sufficiently small in size, they will propagate through the tiniest vascular channels (capillary beds) without clogging them. Thus, when approximately 30 micron size diamond chips are used, the diamond chips are embedded in the cutting head so that when the diamond chips contact tissue at a velocity of about 12.2 m/sec the tissue fragments so produced can easily be less than 5 microns, i.e., less than the size of a red blood cell, which, of course, propagates through the capillary network. A 5 micron size debris fragment contains many millions of constituent molecules. Accordingly, the energy required to produce such fragments is orders of magnitude less than would be required if a laser using molecular evaporation was employed. Releasing many calories of energy within a blood vessel (using a laser) carries an attendant high risk of vessel wall damage by thermal conduction and subsequent thermal necrosis.

In accordance with a preferred embodiment of the present invention, an elliposoidal cutting head similar in shape to that depicted in U.S. Patent No. 4,445,509 is used, which is coated preferrably with tiny diamond chips (shovels). The cutting head is rotated at a speed which, in conjunction with its geometrical circumference, provides a surface velocity of about 12.2 m/s approximately 40 ft/sec). It has been found that a tip of this type, operated at such a tip velocity, is able to cut soft material at a high removal rate, while generating microscopic particles (on the order of 5 microns or less) and leaving behind a tissue base having a smooth appearance. Such tips can now be fabricated in sizes ranging from about .5 mm in diameter up to 6 mm diameter. To achieve a surface speed of 12.2 m/s (40 ft/sec) with a tip of 1.5 mm diameter requires a rotation rate of approximately 155,000 revolutions per minute (rpm).

Transmission of such high rates of rotation through a flexible catheter has recently been shown to be possible using a 0.51 mm (.020") trifilar helically wound drive shaft spinning within a thin plastic tube using a 0.127 mm (.005") diameter solid steel shaft as a stationary core or rail. Infusion of biocompatible saline through the plastic sheath provides cooling of the sliding interface during operation.

Using the same mechanical configuration but operating at reduced rpm allows the same device to preferentially cut hard material while sparing soft material. Operation at high rotation speed will, of course, cut hard material very well. Indeed, hard material is usually removed more easily at all speeds relative to soft material. The point is that at very high surface speeds (about 12.2 m/s (approximately 40 ft/sec) and above) even the soft tissue can be cut, whereas at lower speeds it is very difficult to remove, but the hard material can still be dissected. Thus, a single device whose speed is modulated becomes a multipurpose device capable of differential cutting or soft tissue cutting. This device has now been shown to work in a variety of animal tissues varying from soft to hard while being flexibly conveyed through a plastic catheter.

In the Drawing:
FIG. 1 depicts a preferred embodiment of the present invention; and
FIG. 2 is an exploded side view of the tip of the device of FIG. 1.

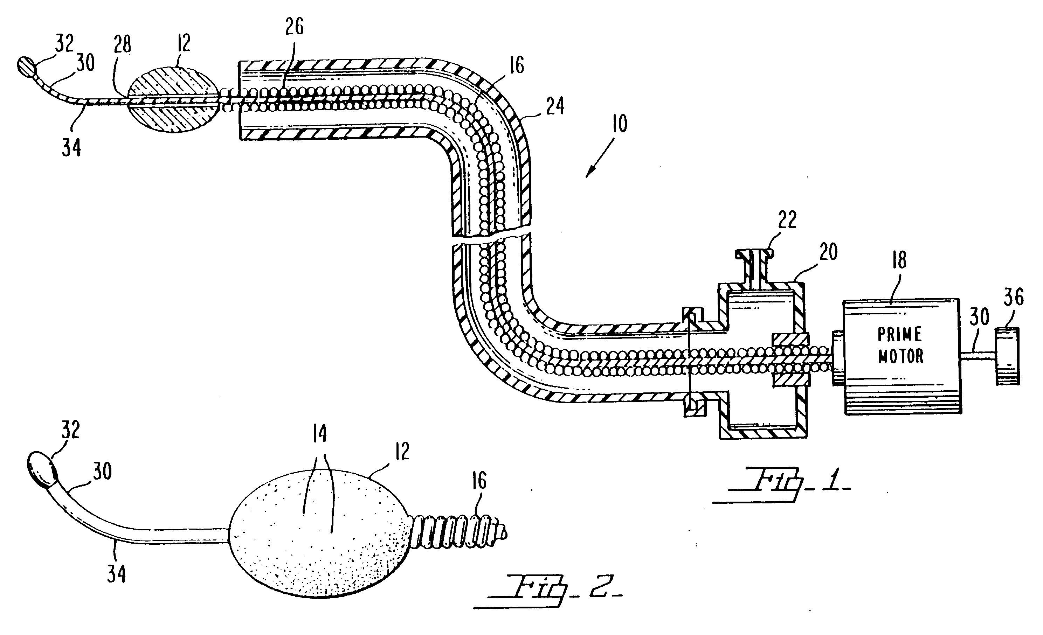

Referring generally to FIGS. 1 and 2, the preferred embodiment 10 of the present invention is shown. The preferred embodiment 10 comprises an abrasive tip 12 and an atraumatic tip 32 which is generally of the type described in EP-A-177 782 entitled TRANSLUMINAL THROMBECTOMY APPARATUS, of the present inventor which is steerable for accessing branch vessels. The tip 12 is covered with an abrasive cutting material, such as diamond grit 14, which is used in the preferred embodiment of the invention. The tip 12 is connected via a hollow, flexible drive shaft 16 to a variable speed prime mover 18. In the preferred embodiment of the invention, the drive shaft 16 is a 0.51 mm (.020") diameter trifilar helically wound drive shaft. The drive shaft 16 is sealably coupled to a variable speed rotational prime mover 18, which is capable of high speed rotation. The coupling is accomplished using a sealed chamber 20 having an injection port 22, so that injection of drugs or fluids into the lumen which is formed between the drive shaft 16 and a surrounding plastic sheath can be accommodated. The distal segment 26 of the flexible shaft 16 is preferrably passivated with a coating, of a material such as PTFE, which will inhibit the winding of intravascular fiber on the shaft 16 during rotation. The tip 12 includes a central bore 28 which is aligned with the opening which extends down the length of the hollow shaft 16. The tip 12 and the shaft 19 are routed into a vessel by using a central guide rail 30, which may be comprised of 0.127 mm (.005") diameter steel wire. Adjacent the blunt tip 32 at the distal end of the guide rail 30, there is a preformable portion 34 of the guide rail 30 which the physician using the invention may bend to facilitate directing the invention into branch vessels. The guide rail 30 extends completely through the shaft 16 and through the prime mover 18 to a rotatable knob 36 which permits the guide rail 30 to be rotated in order to direct the tip 32 through a patient's vessel in order to perform a thrombectomy as described in U.S. patent 4,679,557. The drive shaft 16 and the central rail 30 may be individually moved with respect to each other and with respect to the plastic sheath 24 in order to engage a thrombus or an atheromatous occlusion. The rotational prime mover 18 for the high-speed helical drive shaft 16 is preferably operable in a range of from 20,000 rpm to greater than 155,000 rpm. The size of the burr tip 12 is typically in a range of from less than 1 mm diameter up to about 6 mm, depending upon the lumen size desired in the lesion being recanalized.

Such a device provides for transluminal recanalization of intravascular lesions of soft or hard constitution consisting of thrombotic or atheromatous material.

**Claims**

1. A transluminal microdissection device comprising:
   a) a rotatable flexible drive shaft (16) having a cutting tip (12) attached thereto, said tip (12) having a diameter which is greater than that of said drive shaft (16); and
   b) an abrasive surface on said tip (12), characterized in that said abrasive surface is comprised of an abrasive material (14) attached to the surface of said tip (12).

2. The transluminal microdissection device of Claim 1 wherein said drive shaft (16) and said tip (12) are hollow, whereby they may be guided along a guide wire (30).

3. The transluminal microdissection device of Claim 1 or 2 further comprising a rotatable prime mover which is capable of rotating said drive shaft (16) at a rotation rate of from about 20,000 revolutions per minute to at lest 155,00 revolutions per minute.

4. The transluminal microdissection device of Claims 1 to 3 further comprising a tubular sheath (24) which surrounds said flexible drive shaft (16) and chamber means (20) for sealably attaching to said tubular sheath (24), said chamber means (20) including a seal through which said drive shaft (16) passes.

5. The transluminal microdissection device of Claims 1 to 4 further comprising a port (22) which extends into said chamber means (20) whereby the lumen formed between said drive shaft (16) and said tubular sheath (24) is accessible through said port (22).

6. The transluminal microdissection device of claims 1 - 5, wherein said guide wire (30) comprises a tip (32) at its distal end.

7. The transluminal microdissection device of claim 6, wherein the said guide wire (30) comprises a preformable portion (34) adjacent to said tip (32) at its distal end.

8. A transluminal microdissection device of claims 1 - 7, wherein said abrasive material (14) is comprised of crystalline fragments, preferably fragments of diamond crystal or grit.

9. The transluminal microdissection device of Claim 8, wherein said crystalline fragments have a size of approximately 30 microns, so as to produce tissue fragments having a size preferably less than 5 microns.

**Revendications**

1. Dispositif transluminal de microdissection comprenant:
   a) un arbre d'entraînement souple tournant (16) auquel est fixée une pointe coupante (12), ladite pointe (12) ayant un diamètre supérieur à celui dudit arbre d'entraînement (16); et
   b) une surface abrasive sur ladite pointe (12), caractérisé en ce que ladite surface abrasive est formée d'une matière abrasive (14) fixée à la surface de ladite pointe (12).

2. Dispositif transluminal de microdissection selon la revendication 1, dans lequel ledit arbre d'entraînement (16) et ladite pointe (12) sont creux, ce qui permet de les guider le long d'un fil de guidage (30).

3. Dispositif transluminal de microdissection selon la revendication 1 ou 2, comprenant en outre un appareil d'entraînement tournant qui est capable de faire tourner ledit arbre d'entraînement (16) à une vitesse de rotation d'environ 20 000 tours/mn à au moins 155 000 tours/mn.

4. Dispositif transluminal de microdissection selon les revendications 1 à 3, comprenant en outre une gaine tubulaire (24) qui entoure ledit arbre d'entraînement souple (16), et une chambre (20) destinée à être fixée de manière étanche à ladite gaine tubulaire (24), ladite chambre (20) comportant un joint d'étanchéité que traverse ledit arbre d'entraînement (16).

5. Dispositif transluminal de microdissection selon les revendications 1 à 4, comprenant en outre un orifice (22) qui donne dans ladite chambre (20) pour que le canal formé entre ledit arbre d'entraînement (16) et ladite gaine tubulaire (24) soit accessible par ledit orifice (22).

6. Dispositif transluminal de microdissection selon les revendications 1 à 5, dans lequel ledit fil de guidage (30) comprend un bout rond (32) à son extrémité distale.

7. Dispositif transluminal de microdissection selon la revendication 6, dans lequel ledit fil de guidage (30) comprend une partie préformable (34) près dudit bout rond (32) à son extrémité distale.

8. Dispositif transluminal de microdissection selon les revendications 1 à 7, dans lequel ladite matière abrasive (14) est composée de fragments cristallins, de préférence des fragments de cristaux ou de particules de diamant.

9. Dispositif transluminal de microdissection selon la revendication 8, dans lequel lesdits fragments cristallins ont une taille de 30 microns environ, de manière à former des fragments de tissus dont la taille est de préférence inférieure à 5 microns.

**Patentansprüche**

1. Transluminale Mikrosektionsvorrichtung mit:
   a) einer drehbaren flexiblen Antriebswelle (16) mit einer daran angebrachten Schneidspitze (12), wobei die Spitze (12) einen Durchmesser hat, der größer ist als der der Antriebswelle (16), und
   b) einer Abreibfläche an der Spitze (12),
   **dadurch gekennzeichnet,** daß die Abreibfläche aus einem an der Fläche der Spitze (12) angebrachten Abreibmaterial (14) gebildet wird.

2. Transluminale Mikrosektionsvorrichtung nach Anspruch 1, wobei die Antriebswelle (16) und die Spitze (12) hohl sind, wodurch diese entlang eines Führungsdrahts (30) geführt werden können.

3. Transluminale Mikrosektionsvorrichtung nach Anspruch 1 oder 2, ferner mit einem drehbaren Hauptantrieb, der geeignet ist, die Antriebswelle (16) mit einer Drehgeschwindigkeit von etwa 20 000 UpM bis mindestens 155 000 Umdrehungen/Min. zu drehen.

4. Transluminale Mikrosektionsvorrichtung nach Anspruch 1 bis 3, ferner mit einer rohrförmigen Hülle (24), die die flexible Antriebswelle (16) umgibt, und einer Kammereinrichtung (20) zum dichtenden Anbringen der rohrförmigen Hülle (24), wobei die Kammereinrichtung (20) eine Dichtung aufweist, durch die die Antriebswelle (16) hindurchgeht.

5. Transluminale Mikrosektionsvorrichtung nach Anspruch 1 bis 4, ferner mit einem Anschluß (22), der sich in die Kammereinrichtung (20) erstreckt, wodurch das zwischen der Antriebswelle (16) und der rohrförmigen Hülle (24) gebildete Lumen durch den Anschluß (22) zugänglich ist.

6. Transluminale Mikrosektionsvorrichtung nach Anspruch 1 bis 5, wobei der Führungsdraht (30) an seinem distalen Ende eine Spitze (32) aufweist.

7. Transluminale Mikrosektionsvorrichtung nach Anspruch 6, wobei der Führungsdraht (30) benachbart zu der Spitze (32) an seinem distalen Ende einen vorformbaren Abschnitt (34) aufweist.

8. Transluminale Mikrosektionsvorrichtung nach Anspruch 1 bis 7, wobei das Abreibmaterial (14) aus kristallinen Teilchen, vorzugsweise Teilchen von Diamantkristallen oder -körnern gebildet wird.

9. Transluminale Mikrosektionsvorrichtung nach Anspruch 8, wobei die kristallinen Teilchen eine Größe von ungefähr 30 $\mu$m haben, so daß Gewebeteilchen mit einer Größe von vorzugsweise < 5 $\mu$m erzeugt werden.

Fig_1_

Fig_2_

EP 0 229 620 B1